# EUROPEAN PATENT SPECIFICATION

(11) **EP 1 096 964 B1**
(45) Date of publication and mention of the grant of the patent: **20.08.2003**
(21) Application number: 99934837.8
(22) Date of filing: 09.07.1999
(51) Int. Cl.: A61M 16/06

(54) **BREATHING MASKS**
ATEM-MASKEN
MASQUES A OXYGENE

(30) Priority: 10.07.1998 GB 9814890; 20.11.1998 GB 9825365
(43) Date of publication of application: 09.05.2001
(73) Proprietor: Respitec Company Limited, Blundellsands, Liverpool L23 6SN (GB)
(72) Inventor: TOWNSEND-ROSE, Chris R, 1 Burbo Bank Road South, Liverpool L23 6SN (GB); BREAKELL, Andrew, Liverpool L38 9GE (GB)
(74) Representative: Collingwood, Anthony Robert
(86) International application number: GB9902215
(87) International publication number: WO00002611

(56) References cited:
- EP-A- 0 234 746
- WO-A-92/17146
- GB-A- 649 689
- US-A- 4 150 671
- US-A- 4 417 589
- US-A- 4 878 502

## Description

Oxygen masks and other types of breathing apparatus for supplying a specified gas or gas mixture are widely used. For example, such masks are employed routinely in hospitals, by the emergency services, and on passenger aircraft for supplying oxygen to an individual who is having breathing difficulties. It is, however, difficult to ensure that the mask is attached properly to the subject . If an oxygen mask is fitted incorrectly, a seal may not form between the mask and the face of the subject, thereby allowing oxygen gas to escape form the mask rather than being available to the subject. Such an escape of gas is costly and could be damaging to the subject's wellbeing, and furthermore may not be noticeable upon visual inspection of currently available oxygen masks. A study by Milross and co-workers (Milross, J., I. H. Young, P. Donnelly, 1989, Anaesth. Intensive Care 17(2): 180-184) has shown that the commercially popular Hudson oxygen mask has an inspired oxygen fraction (i.e. percentage oxygen delivery) of 78% if the mask is well fitted but only 46% if the mask is loosely fitted. There is clearly a need for a device which indicates the efficiency of the face to mask seal.

Currently available masks do not provide an indicator of gas supply flow. Normally indicators of flow are separated from the mask and may be found against a side wall and/or on gas cylinders beneath a patient's trolley. If the oxygen supply, for example, becomes disconnected or the supply tubing is kinked such that oxygen flow to the patient is impeded, it is not possible to ascertain the disruption whilst looking at the patient. A device that indicates oxygen flow through the mask itself would therefore represent a substantial improvement over available systems.

Respiratory arrest (i.e. cessation of breathing) in a subject is difficult for a person to diagnose without suitable equipment. Chest movements of the subject can be observed, and the sound of breathing at the mouth monitored, over a length of time. With shallow breathing, chest movements are minimal and therefore difficult to hear or count, especially if the subject is clothed.

Medical practioners, in particular, are frequently required to record the respiratory rate of a patient. In cases where patients are acutely ill with respiratory diseases or after trauma, their respiratory rate can be correlated with their medical progress. Respiratory rate is routinely monitored during and after surgery. In the absence of sophisticated equipment, and in most cases, respiratory rate is often calculated after visual determination of chest movements over a period of time. Such a method may be difficult for reasons mentioned *supra*, and is frequently inaccurate.

US 4 878 502 (Dietz) discloses a device which incorporates a breathing sensor that monitors inhalation and exhalation of air by sensing the flow of air into a patient's nostril. The device relies upon inspiration to draw up a floating ball in a tube, against gravity, to monitor breathing of the patient. US 4 417 589 (Favaloro) discloses a system which monitors the breathing of mammals (human and non-human). Favaloro's system contains a gas flow detector tube in which the positioning of a ball responding to the mammal's breath can allow the triggering of an alert signal when breathing ceases.

Other known breathing apparati include: a face-protective weathermask (US 4 150 671); a valve-controlled inhalation apparatus (GB 649689); and a portable positive pressure emergency respirator (WO 92/17146).

Both US 4 878 502 and US 4 417 589 mentioned *supra* disclose invasive devices which may be uncomfortable to use. These devices may also not have sufficient flexibility to detect two extremes of breathing, i.e. light, slow, shallow breathing versus fast, deep, forceful breathing, without adjustments. Furthermore, such devices are not suitable for roadside or remote use, and are too expensive for wide distribution. Neither of the devices detailed *supra* are designed for determining whether a face mask is correctly fitted and supplying oxygen to the subject. Nor do these devices address problems associated with prolonged use of masks, such as the accumulation of water vapour.

The present invention addresses problems associated with the prior art and provides an improved device that allows monitoring of the mask fit and seal, supply of gas flow, breathing and respiration rate, yet is non-invasive, inexpensive, durable, lightweight, safe and easy to use.

According to the present invention there is provided a breathing mask having a housing communicating with the interior of the mask and extending outwardly therefrom, an opening in the housing remote from the mask and a member in the housing movable in response to gas flow through the housing.

The movable member in the housing of the breathing mask may comprise a ball, and said ball may be treated with a chemical to reduce electrostatic forces. The movable member may aslo be in the form of a disk. The movable member may be manufactured from lightweight materials, such as polystyrene, so that it is sensitive to small deviations in gas or air flow.

The housing of the breathing mask may permit sight of the movable member through its wall. The housing may be made of a plastic material and may be sufficiently transparent so as to allow visibility of the movable member.

The breathing mask may include means for automatically monitoring movement of the movable member. Said means may include an infra-red emitter and receiver disposed on opposite sides of the housing.

The housing may comprise a tubular duct. Said duct may be of L-shape, the first of whose limbs communicates with the mask and the second of whose limbs contains the movable member. Furthermore, the first limb communicating with said mask may be rotatable through 180 degrees or more. The gas supply to the mask may also supply gas to the second limb of the L-shaped duct, and said gas supply to the second limb may be shut off. The second limb may extend upwardly when gas is supplied thereto and downwardly when it is not.

The breathing mask may include a one-way valve at the communication between the mask and the housing to prevent passage of gas from the mask to the housing. The one-way valve prevents air from entering the housing and allowing condensation to form, which would interfere with the operation of the device. A water-absorbent material, such as silica gauze, may also be contained within the device.

The breathing mask is provided with ventilation holes to allow expulsion of exhaled air, as is standard in face masks.

The breathing mask may be disposable after use.

Also provided according to the present invention is an attachment for a breathing mask comprising a housing having spaced openings therein, means for attaching the housing to a breathing mask so that one of said openings communicates with the interior of the mask and a member in the housing movable in response to gas flow through the housing.

A friction-fit arrangement, a screw-thread and a coupling sleeve are examples of means for attaching the housing to the breathing mask, the breathing mask being provided with an appropriate fitting for the attachment. For example, there may be an opening on either or both sides of the nose-piece of the mask.

The attachment device of the present invention could be attached to any type of breathing apparatus, including nasal air delivery tubes, paediatric masks, gas masks, smoke hoods, breathing filters, catheter mounts and resuscitation bags. The device may also be attached to, or be integral with, a life saver pocket mask.

The movable member of the attachment may comprise a ball, which if liable to become electrostatically charged, may be coated with an antistatic agent.

The housing of the attachment may permit sight of the movable member through its wall.

The attachment may include means automatically to monitor movement of the movable member. Said means may include an infra-red emitter and receiver disposed on opposite sides of the housing. The monitoring means could be connected to an electronic counter for displaying the respiration rate of the patient, eg. via a liquid crystal display (LCD) unit. The counter could be a small pocket-sized electronic device for attachment via a clip or other means to the subject and may be linked to a computer monitor. The means to monitor movement of the movable member may be linked to an alarm (visual or aural) which is activated when the respiration falls below or exceeds a predetermined threshold.

The housing of the attachment may comprise a tubular duct. Said duct may be of L-shape, the first of whose limbs is adapted to communicate with the mask and the second of whose limbs contains the movable member. The first limb, communicating with said mask, may be rotatable through 180 degrees or more. The attachment may include pipe means enabling a portion of gas supply to the mask to enter said second limb. The attachment may be adjustable when attached to the mask to enable the second limb to extend either upwardly when gas is supplied thereto or downwardly when it is not.

The attachment may include a one-way valve to prevent gas entering the housing from the interior of the mask.

The attachment may be disposable after use.

The invention will be further apparent from the following description, with reference to the several figures of the accompanying drawings, which show, by way of example only, various mask devices and their operation.
- Figure 1: is a schematic diagram of the separate components of a mask and housing according to one embodiment of the present invention, indicating how the housing slides into the mask;
- Figure 2: is a schematic diagram of the components of Figure 1 attached, showing oxygen running through an unused mask;
- Figure 3: shows the device of Figure 2 linked to an LCD monitor;
- Figure 4: is a schematic diagram of the device of Figure 2 in the upright position, during exhalation;
- Figure 5: is a schematic diagram of the device of Figure 2 in the upright position, during exhalation;
- Figure 6A: is a schematic diagram of the device of Figure 2 in the downward position, as it appears in the absence of breathing and with no oxygen flow to the mask;
- Figure 6B: is a schematic diagram of the device of Figure 2 in the downward position, as it appears during inhalation, but with no oxygen flow to the mask;
- Figure 7: shows a face mask according to a further embodiment of the invention;
- Figure 8: is a section through part of the face mask of Figure 7;
- Figure 9A and B: show schematically a first variation of the face mask of Figures 7 and 8 respectively;
- Figure 10: shows schematically a second variation of the face mask of Figures 7 and 8; and
- Figure 11: shows schematically a third variation of the face mask of Figures 7 and 8.

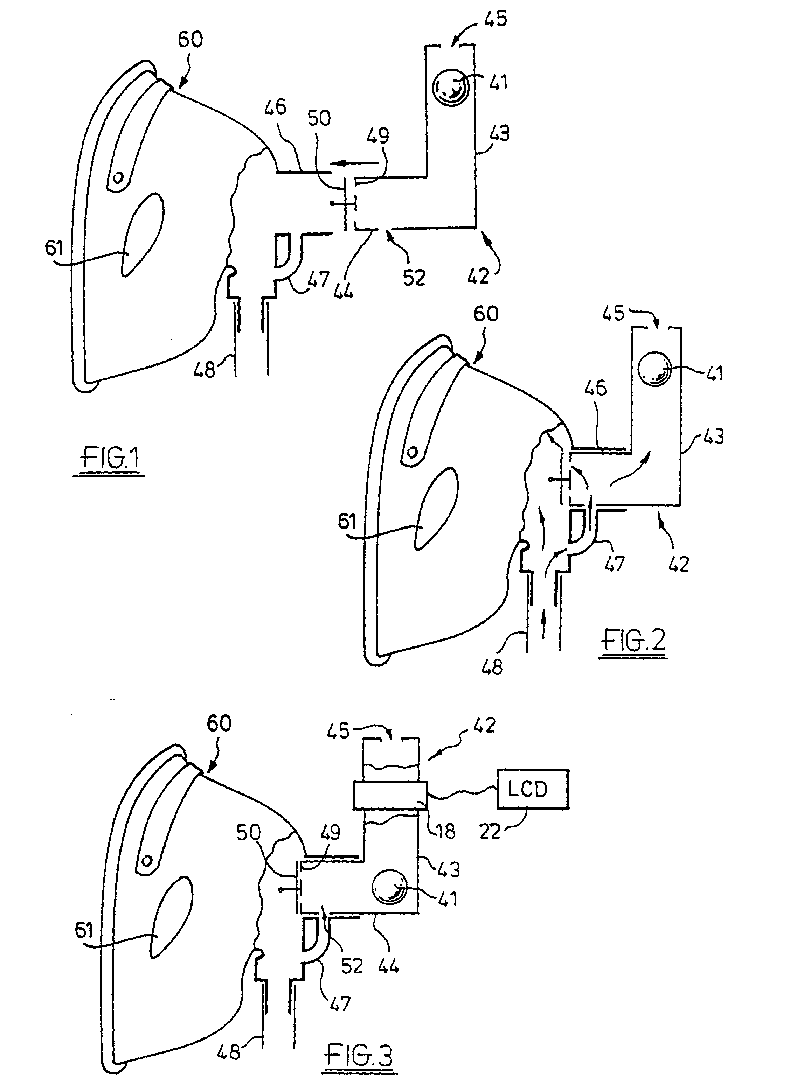

Figure 1 of the accompanying drawings illustrates the housing and mask of one embodiment of the present invention and how the housing and mask are connected together. The device comprises an L-shaped transparent cylindrical tube 42 with a short arm 44 and a long arm 43. The tube 42 is provided with an aperture 45 at one end, an opening 52 in the short arm 44, a perforated end plate 49 to the short arm 44 and a one-way valve 50. A lightweight ball 41, preferably made of polystyrene, is retained within the tube. Polystyrene may in certain conditions become electrostatically charged, but the ball can be treated with an antistatic agent. The tube is made of a plastics material; other suitable materials are also in the scope of the present invention. The ball 41 is dimensioned to be freely movable within tube 42, but is too large to exit through aperture 45. The opening 52 of the short arm 44 allows the flow of a gas into the tube 42 when the tube 42 and mask 60 are connected (see Figure 2, *infra*). The free end of the short arm 44 is provided with a one-way valve 50 which allows air flow out of the tube 42 through opening 52, but will not allow flow in the opposite direction i.e. from mask 60 to tube 42. The mask 60 is provided with a forwardly extending portion which forms a sleeve 46 for receiving tube 42, and ventilation port holes 61 for the escape of expired gases. The inner circumference ofthe sleeve is only slightly larger than the outer circumference of the tube 44 to allow the tube to be rotated therein with the application of pressure, thereby acting in a similar manner to a plug cock or tap. A supplementary oxygen delivery tube 47 delivers oxygen to the tube 42 via the opening 52. The supplementary delivery tube 47 is a branch from the main oxygen supply through delivery tube 48 supplying the mask 60.

The provision of the one-way valve 50 prevents the exhaled air from entering the tube 42. The exhaled air is expelled through a ventilation port 61 provided in the mask 60. Valve 50 is an important feature of the present invention because exhaled air contains water vapour which could condense in the tube 42 and affect functioning of the movable member. The tube 42 may be flexible or provided with a region of concertinas to allow the tube 42 to be moved into the appropriate position regardless of the position of the subject.

Tube 42 may alternatively be any shaped conduit or vessel for containing a movable member, and having an aperture to allow entry of air. It is also to be appreciated that the tube 42 does not have to be entirely transparent but may have only a transparent section to allow visibility of the ball 41. The aperture 45 may be provided by rolled edges moulded integrally with the tube 42 or may be in the form of a perforated disc for attachment over the end of the tube 42.

Figure 2 of the accompanying drawings shows an embodiment of the present invention when the supply of oxygen to the mask is on but the mask is not connected to the patient. Oxygen will flow as directed by the arrows and the ball 41 will be in the upper part of the tube 42. If the mask 60 is not attached to a patient, the position of the ball 41 can alert personnel that the oxygen supply has not been turned off.

The device shown in Figures 1 and 2 may also be used to measure the rate of respiration of an individual simply and accurately by counting the number of times the ball 41 rises and falls within the tube 42 over a given period of time. When the patient exhales, the ball 41 moves towards aperture 45 of the tube 42 by virtue of the flow of gas. In this manner, the respiration rate per minute may be determined by, for example, counting the number of downstrokes of the ball 41 over 15 seconds (e.g., 3 strokes over 15 seconds would indicate a respiration rate of 12 breaths per minute). This is easier and more accurate than trying to measure the chest movement of an individual over a given period of time.

The device of the present invention may also include means for automatically counting and recording the number of strokes of the ball when the device is attached to a subject, thereby providing an automated system for monitoring the respiration rate of the individual. Figure 3 of the accompanying drawings shows an embodiment of the invention having a ring 18 fitted over the tube 42. An infra red transmitter and receiver within ring 18 detect the number of times the ball 41 breaks the beam as it passes up and down the long arm 43. The ring 18, preferably has an inner circumference just slightly greater than the outer circumference of the long arm 43. The sensed rate of respiration is displayed on a liquid crystal display (LCD) monitor 22.

The counter may also be connected to an alarm (not shown) which is activated when the respiration rate falls below and/or exceeds a predetermined lower and upper limit (eg. < 10 min⁻¹ or > 40 min⁻¹), thereby acting as an apnoea or hyperpnoea/tachypnoea alarm, respectively. The alarm may be visual and/or audible.

For the purposes of this description, we define "upward position" of the device when the long arm 43 of the tube 42 has aperture 45 opening outwardly away from the ground, such that a movable member within tube 42 naturally falls away from the aperture 45 under the influence of gravity. In a "downward position", aperture 45 opens outwardly towards the ground, such that a movable member within tube 42 will fall towards aperture 45 under the influence of gravity.

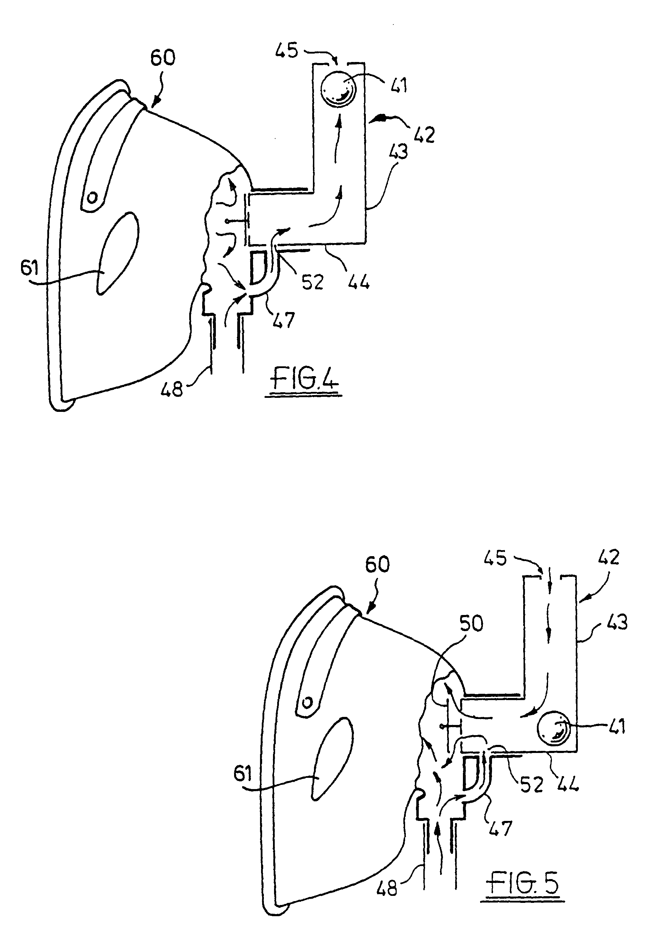

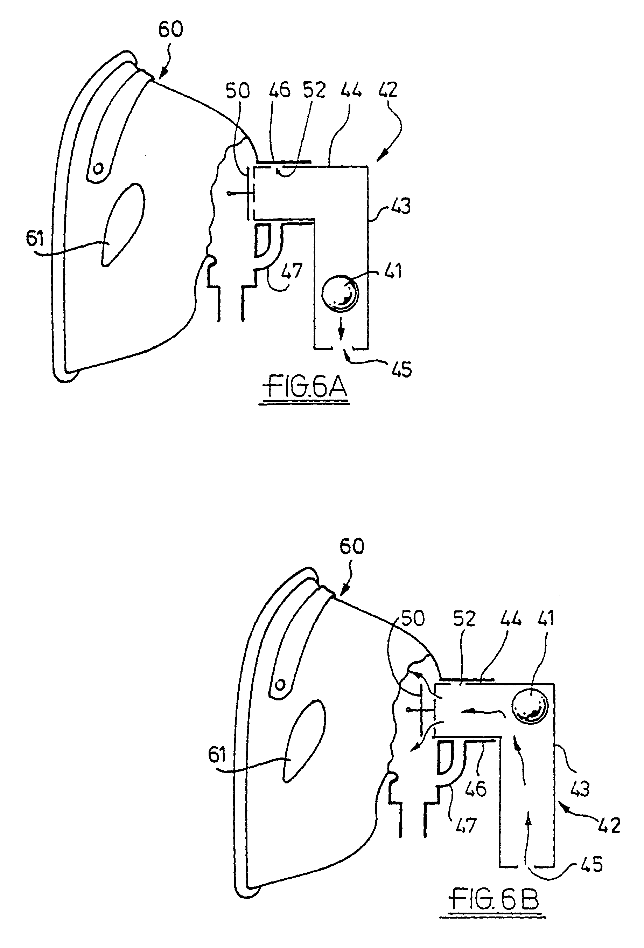

The device has two operating positions: an upward position (Figures 4 and 5) and a downward position (Figure 6A, B). In the upward position, opening 52 of the short arm 44 communicates with the supplementary delivery tube 47, i.e., the tap is in the open position. Oxygen flowing into the mask 60 is able to pass into the tube 42 through the opening 52. This will cause the ball 41 in the long arm 43 of the tube to rise towards the aperture 45 of the tube 42, thereby demonstrating that oxygen is flowing to the mask 60 (Figure 4).

It is also possible to check a subject's breathing rate. With the device in the upward position, the ball 41 will move up and down within the tube 42 in response to the flow of oxygen and inspiration of the subject, respectively (Figure 5). Exhaled air cannot enter the tube 42 due to the provision of the one-way valve 50, and is expelled through ventilation holes 61 provided in the mask 60. If the mask to face seal is inadequate, the ball 41 will not move within the long arm 43 in response to breathing.

If the subject does not require a supply of oxygen, the device positioned as shown in Figure 6 may still be used to check that the subject is breathing and to determine the respiration rate. The tube 42 is positioned such that the device is in a downward position. In this position, the opening 52 and the supplementary delivery tube 47 are no longer in communication (i.e. the tap is shut) and ball 41 falls towards the aperture 45 of the long arm 43 under the influence of gravity. If the subject is breathing, the ball 41 will rise up the long arm 43 on inhalations as a result of air being sucked in through the aperture 45. Exhaled air is again prevented from entering the tube 42 by the one-way valve 50 and thus the ball 41 falls to the base of the tube 42 during exhalation. Respiration rate may be conveniently and accurately measured by counting the number of upstrokes of the ball 41.

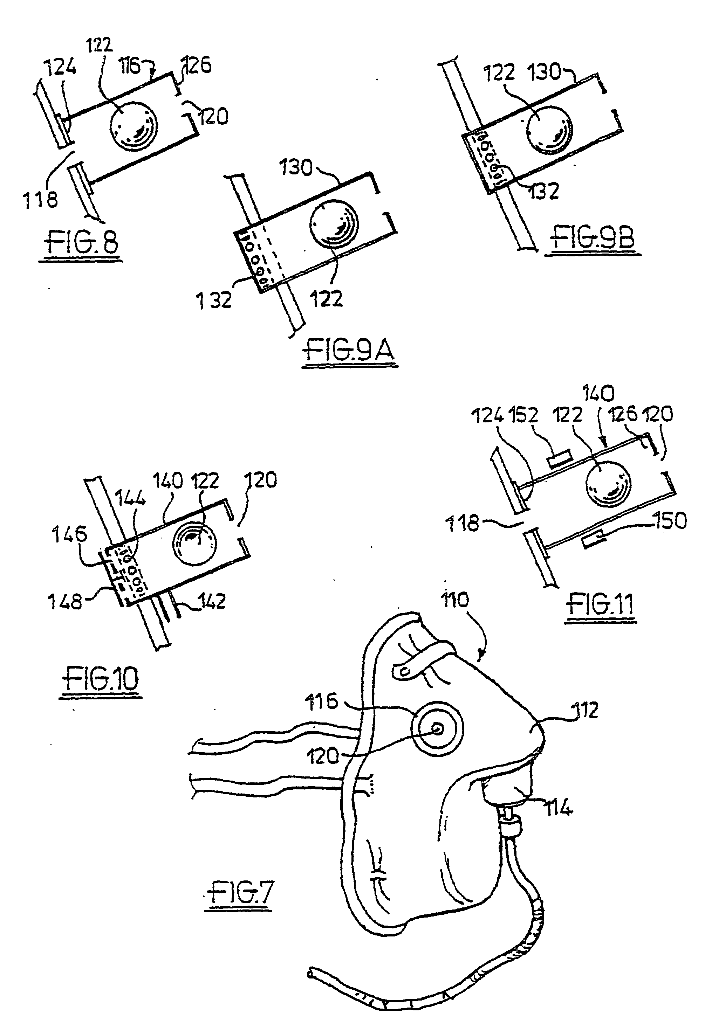

A further embodiment of the present invention is illustrated in Figures 7 to 11. Referring to Figures 7 and 8, a face mask 110 for supplying oxygen comprises a plastics enclosure 112 shaped to fit over the patient's nose and mouth. An oxygen inlet 114 is provided at the front of the mask 110 below the enclosure 112. To one side of the mask 110 there is provided a cylindrical housing 16 extending outwardly therefrom. The housing 110 has an aperture 118 (see Figure 8) at one end communicating with the interior of the mask 110 and an aperture 120 at its other end remote from the mask 110. The housing 116 is of transparent plastics material and contains a light weight ball 122, which is free to move back and forth in the housing but cannot escape therefrom past retaining lips 124, 126 at opposite ends of the housing 116.

The mask 110 operates in the following manner. When oxygen is supplied to the mask, the ball will be forced to the remote end of the housing 116 (ie. towards opening 122). However, provided the patient is breathing, each intake of breath will reduce the pressure inside the mask 110 and cause the ball 122 to move towards the mask 110, moving back out again before the next intake. Detection of such movement of the ball 122 indicates breathing activity. The rate of movement of the ball 122 can be monitored to give a respiration rate for the patient.

On the other hand, if the mask 110 is not fitted correctly, so that oxygen is leaking from sides of the mask 110, the pressure inside the mask 110 will reduce allowing the ball 122 to move back towards the mask 110 and stay there, thus indicating poor fitting of the mask 110.

Figures 9A and B illustrate a variation in which the breathing mask 110 can be used without respiration detection. The housing 130 has apertures 132 around its outer surface for communication with the interior of the mask (not shown) instead of the single aperture 118 of Figure 8. The housing 130 is movable between two positions, so that, in the position shown in Figure 9A, the apertures 132 are open to the interior of the mask for respiration detection, while in the position shown in Figure 9B, the apertures 132 are closed by the surrounding mask, so that respiration is not detected.

A second variation, shown in Figure 10, has a housing 140 with an oxygen supply inlet 142. This is in place of an oxygen inlet to the mask *per se*. The housing 140 has at its end communicating with the interior of the mask not only a plurality of apertures 144 around its periphery enabling it to be used in two positions as described in relation to the variation of Figure 9, but also apertures 146 in its end communicating with the interior of the mask closed by a one-way valve 148.

Thus, in both positions of housing 140, oxygen can continue to be supplied to the mask via the apertures 146 and the one-way valve 148. By sliding the housing into the mask, so that the apertures 144 are open to the interior of the mask breathing activity of the patient can be detected by movement back and forth of the ball 122.

In Figure 11, there is a variation shown with reference to the embodiment of Figure 8 but equally applicable to any other embodiment of the invention. Whilst movement of the ball 122 in the housing 140 is detectable visually, it may be desirable, independently to detect and record the movement of ball 122. To that end, an infra red emitter 150 is positioned on one side of housing 116 and an infra red detector 152 on the opposite side. The emitter 150 emits out a beam that is detected by the detector 152 except when it is interrupted by passage of the ball 122 due to respiration activity. The output from the detector thus corresponds with respiration activity, and can be monitored electronically and recorded. An alarm condition indicator may be linked to the signal detection, whereby absence of signals due to lack of respiration activity for a predetermined period of time produces an alarm signal.

Life saver pocket masks are supplied in the majority of first aid kits and are often used by people having minimal or no experience in resuscitation techniques. The present invention allows a user, firstly, to establish whether the subject is breathing and, secondly, to determine the respiration rate of the subject. If the subject resumes spontaneous and independent respiration this will be immediately evident to the life-saver because the movable member will be moving independently of his efforts during inhalation and hence, assisted respiration may be ceased accordingly. If assisted breathing is required, the provision of a one-way valve in the device prevents the air exiting the pocket mask through the device rather than passing into the individual. When an ambulance arrives, oxygen can be connected to the mask, the tube turned into the upright position and respiratory monitoring can commence.

It is evident that the present invention provides an hygienic and simple device for improving the safety aspects of medical or other oxygen delivery masks, and for indicating respiration and measuring, either manually or automatically, respiration rate. The device enables medical staff to monitor the respiratory rate at a fraction of the cost of devices which measure carbon dioxide. The device also provides a simple means for determining whether a breathing mask has been fitted correctly and is delivering an unobstructed flow of a gas, thereby safeguarding against the gas supply being cut off or the apparatus becoming loose. There is thought to be, currently, no device which verifies that oxygen is being delivered to a mask. The device can also indicate that the mask to face seal is satisfactory and that the patient is receiving a prescribed amount of oxygen. Over - or under - supply can be dangerous. Leakage of oxygen can be a fire hazard, and is also expensive. The provision of a one-way valve allows the uni-directional flow of gas and prevents condensation build up.

The device is non-invasive, having no components which require insertion into the patient's nostrils or mouth. Devices according to the invention may be fully disposable and may be adapted to be used without an oxygen supply by a simple adjustment. In this mode a device could aid in bystander resuscitation, e.g. to check whether a person is breathing after resuscitation, or not breathing, indicating a need to begin resuscitative procedures.

## Claims

1. A breathing mask (60) having a housing (42) communicating with the interior of the mask and extending outwardly therefrom, an opening (45) in the housing remote from the mask, **characterised in that** the mask comprises a gas flow indicator member (41) in the housing movable in response to gas flow through the housing.

2. A breathing mask according to claim 1, including a one-way valve (50) at the communication between the mask and the housing to prevent passage of gas from the mask to the housing.

3. A breathing mask according to either one of claims 1 or 2 wherein said gas flow indicator member comprises a ball.

4. A breathing mask according to claim 3 wherein said ball is treated with an antistatic agent.

5. A breathing mask according to any of claims 1-4 wherein said housing permits sight of the gas flow indicator member through its wall.

6. A breathing mask according to any one of claims 1-5 including means (22) automatically to monitor movement of the gas flow indicator member.

7. A breathing mask according to claim 6 wherein said means includes an infra-red emitter and detector disposed on opposite sides of the housing.

8. A breathing mask according to any preceding claim wherein said housing comprises a tubular duct.

9. A breathing mask according to claim 8 wherein the duct is of L-shape, the first of whose limbs (44) communicates with the mask and the second of whose limbs (43) contains the gas flow indicator member.

10. A breathing mask according to claim 9 wherein said first limb communicating with said mask is rotatable through 180 degrees or more.

11. A breathing mask according to either one of claims 9 or 10 wherein a gas supply to the mask also supplies gas to said second limb.

12. A breathing mask according to claim 11 wherein the gas supply to the second limb can be shut off.

13. A breathing mask according to claim 12 wherein said second limb can be adjusted to extend upwardly when gas is supplied thereto and downwardly when it is not.

14. A breathing mask according to any preceding claim wherein said mask is disposable after use.

15. A breathing mask attachment comprising a housing (116) having spaced openings (118, 120, 132, 144, 146) therein, means (124, 126) for attaching the housing to a breathing mask (110) so that one of said openings communicates with the interior of the mask **characterised in that** the attachement comprises a gas flow indicator member (122) in the housing movable in response to gas flow through the housing.

16. An attachment according to claim 15 including a one-way valve (148) to prevent gas entering the housing from the interior of the mask.

17. An attachment according to either of claims 15 or 16 wherein said gas flow indicator member comprises a ball.

18. An attachment according to claim 17 wherein said ball is treated with an antistatic agent.

19. An attachment according to any of claims 15-18 wherein said housing permits sight of the gas flow indicator member through its wall.

20. An attachment according to any one of claims 15-19 including means (150, 152) automatically to monitor movement of the gas flow indicator member.

21. An attachment according to claim 20 wherein said means includes an infra-red emitter and receiver disposed on opposite sides of the housing.

22. An attachment according to any one of claims 15-21 wherein said housing comprises a tubular duct.

23. An attachment according to claim 22 wherein said duct is of L-shape, the first of whose limbs is adapted to communicate with the mask and the second of whose limbs contains the gas flow indicator member.

24. An attachment according to claim 23 wherein said first limb communicating with said mask is rotatable through 180 degrees or more.

25. An attachment according to either one of claims 23 or 24 including pipe means enabling a portion of gas supply to the mask to enter said second limb.

26. An attachment according to claim 25 wherein the gas supply to the second limb can be shut off.

27. An attachment according to either of claims 25 or 26 which is adjustable when attached to the mask to enable the second limb to extend either upwardly when gas is supplied thereto or downwardly when it is not.

28. An attachment according to any one of claims 15-27 wherein said attachment is disposable after use.

## Patentansprüche

1. Atemmaske (60) mit einem Gehäuse (42), das mit dem Inneren der Maske kommuniziert und sich hiervon nach außen erstreckt, einer Öffnung (45) in dem Gehäuse entfernt von der Maske, und einem Gasströmungs-Anzeigeteil (41) in dem Gehäuse, das in Abhängigkeit von der Gasströmung durch das Gehäuse bewegbar ist.

2. Atemmaske nach Anspruch 1, enthaltend ein Einwegventil (50) in der Verbindung zwischen der Maske und dem Gehäuse, um einen Durchgang von Gas von der Maske zu dem Gehäuse zu verhindern.

3. Atemmaske nach einem der Ansprüche 1 oder 2, bei der das Gasströmungs-Anzeigeteil eine Kugel aufweist.

4. Atemmaske nach Anspruch 3, bei der die Kugel mit einem Antistatikmittel behandelt ist.

5. Atemmaske nach einem der Ansprüche 1 bis 4, bei der das Gehäuse eine Sicht auf das Gasströmungs-Anzeigeteil durch seine Wand hindurch zulässt.

6. Atemmaske nach einem der Ansprüche 1 bis 5, enthaltend Mittel (22) zum automatischen Überwachen der Bewegung des Gasströmungs-Anzeigeteils.

7. Atemmaske nach Anspruch 6, bei der die Mittel eine Infrarot-Emissionsvorrichtung und einen Detektor, die sich auf entgegensetzten Seiten des Gehäuses befinden, aufweisen.

8. Atemmaske nach einem der vorhergehenden Ansprüche, bei der das Gehäuse eine Rohrleitung aufweist.

9. Atemmaske nach Anspruch 8, bei der die Leitung L-förmig ist, wobei der erste ihrer Schenkel (44) mit der Maske kommuniziert und der zweite ihrer Schenkel (43) das Gasströmungs-Anzeigeteil enthält.

10. Atemmaske nach Anspruch 9, bei der der erste, mit der Maske kommunizierende Schenkel um 180° oder mehr drehbar ist.

11. Atemmaske nach einem der Ansprüche 9 oder 10, bei der eine Gaszuführung zu der Maske auch Gas zu dem zweiten Schenkel liefert.

12. Atemmaske nach Anspruch 11, bei der die Gaszuführung zu dem zweiten Schenkel abgeschaltet werden kann.

13. Atemmaske nach Anspruch 12, bei der der zweite Schenkel so eingestellt werden kann, dass er sich aufwärts erstreckt, wenn Gas zu ihm zugeführt wird, und nach unten, wenn es nicht zugeführt wird.

14. Atemmaske nach einem der vorhergehenden Ansprüche, bei der die Maske nach Gebrauch wegwerfbar ist.

15. Atemmasken-Vorsatzgerät mit einem Gehäuse (116) mit im Abstand voneinander angeordneten Öffnungen (118, 120, 132, 144, 146) in diesem, Mitteln (124, 126) zum Befestigen des Gehäuses an einer Atemmaske (110) derart, dass eine der Öffnungen mit dem Inneren der Maske kommuniziert, und einem Gasströmungs-Anzeigeteil (122) in dem Gehäuse, das in Abhängigkeit von der Gasströmung durch das Gehäuse bewegbar ist.

16. Vorsatzgerät nach Anspruch 15, enthaltend ein Einwegventil (148), um den Eintritt von Gas in das Gehäuse von dem Inneren der Maske her zu verhindern.

17. Vorsatzgerät nach einem der Ansprüche 15 oder 16, bei dem das Gasströmungs-Anzeigeteil eine Kugel aufweist.

18. Vorsatzgerät nach Anspruch 17, bei dem die Kugel mit einem Antistatikmittel behandelt ist.

19. Vorsatzgerät nach einem der Ansprüche 15 bis 18, bei dem das Gehäuse eine Sicht auf das Gasströmungs-Anzeigeteil durch seine Wand hindurch zulässt.

20. Vorsatzgerät nach einem der Ansprüche 15 bis 19, enthaltend Mittel (150, 152) zum automatischen Überwachen der Bewegung des Gasströmungs-Anzeigeteils.

21. Vorsatzgerät nach Anspruch 20, bei dem die Mittel eine Infrarot-Emissionsvorrichtung und einen Empfänger, die sich auf entgegensetzten Seiten des Gehäuses befinden, enthalten.

22. Vorsatzgerät nach einem der Ansprüche 15 bis 21, bei dem das Gehäuse eine Rohrleitung aufweist.

23. Vorsatzgerät nach Anspruch 22, bei dem die Leitung L-förmig ist, wobei der erste von ihren Schenkeln zur Kommunikation mit der Maske ausgebildet ist, und der zweite ihrer Schenkel das Gasströmungs-Anzeigeteil enthält.

24. Vorsatzgerät nach Anspruch 23, bei der der erste, mit der Maske kommunizierende Schenkel um 180° oder mehr drehbar ist.

25. Vorsatzgerät nach einem der Ansprüche 23 oder 24, enthaltend Leitungsmittel, welche ermöglichen, dass ein Teil der Gaszuführung zu der Maske in den zweiten Schenkel eintritt.

26. Vorsatzgerät nach Anspruch 25, bei dem die Gaszuführung zu dem zweiten Schenkel abgeschaltet werden kann.

27. Vorsatzgerät nach einem der Ansprüche 25 oder 26, welches einstellbar ist, wenn es an der Maske befestigt ist, um zu ermöglichen, dass sich der zweite Schenkel entweder aufwärts, wenn Gas zu diesem geliefert wird, oder abwärts, wenn es nicht geliefert wird, erstreckt.

28. Vorsatzgerät nach einem der Ansprüche 15 bis 27, bei dem das Vorsatzgerät nach Gebrauch wegwerfbar ist.

## Revendications

1. Masque respiratoire (60) ayant un boîtier (42) communiquant avec l'intérieur du masque et s'étendant vers l'extérieur à partir de celui-ci, une ouverture (45) du boîtier située à distance du masque, et un élément indicateur d'écoulement de gaz (41) situé dans le boîtier, mobile en réponse à un écoulement de gaz à travers le boîtier.

2. Masque respiratoire selon la revendication 1, comportant un clapet unidirectionnel (50) au niveau de la communication entre le masque et le boîtier pour empêcher un passage de gaz depuis le masque vers le boîtier.

3. Masque respiratoire selon l'une des revendications 1 ou 2, dans lequel ledit élément indicateur d'écoulement de gaz comporte une bille.

4. Masque respiratoire selon la revendication 3, dans lequel ladite bille est traitée à l'aide d'un agent antistatique.

5. Masque respiratoire selon l'une quelconque des revendications 1 à 4, dans lequel ledit boîtier permet de voir l'élément indicateur d'écoulement de gaz à travers sa paroi.

6. Masque respiratoire selon l'une quelconque des revendications 1 à 5, comportant des moyens (22) pour surveiller automatiquement le déplacement de l'élément indicateur d'écoulement de gaz.

7. Masque respiratoire selon la revendication 6, dans lequel lesdits moyens comportent un émetteur d'infrarouge et un détecteur d'infrarouge disposés sur des côtés opposés du boîtier.

8. Masque respiratoire selon l'une quelconque des revendications précédentes, dans lequel ledit boîtier est constitué d'un conduit tubulaire.

9. Masque respiratoire selon la revendication 8, dans lequel le conduit a une forme de L, dont la première branche (44) communique avec le masque et dont la seconde branche (43) contient l'élément indicateur d'écoulement de gaz.

10. Masque respiratoire selon la revendication 9, dans lequel la première branche communiquant avec ledit masque peut tourner sur 180 degrés ou plus.

11. Masque respiratoire selon l'une des revendications 9 ou 10, dans lequel une alimentation en gaz vers le masque alimente aussi un gaz vers la seconde branche.

12. Masque respiratoire selon la revendication 11, dans lequel l'alimentation en gaz vers la seconde branche peut être coupée.

13. Masque respiratoire selon la revendication 12, dans lequel ladite seconde branche peut être ajustée pour s'étendre vers le haut lorsque du gaz est alimenté vers celle-ci et vers le bas lorsqu'il n'y en a pas.

14. Masque respiratoire selon l'une quelconque des revendications précédentes, dans lequel ledit masque est jetable après utilisation.

15. Fixation de masque respiratoire comportant un boîtier (116) ayant des ouvertures espacées (118, 120, 132, 144, 146) dans celui-ci, des moyens (124, 126) pour fixer le boîtier sur un masque respiratoire (110) de sorte que l'une desdites ouvertures communique avec l'intérieur du masque et qu'un élément indicateur d'écoulement de gaz (112) situé dans le boîtier peut se déplacer en réponse à un écoulement de gaz à travers le boîtier.

16. Fixation selon la revendication 15, comportant un clapet unidirectionnel (148) pour empêcher qu'un gaz ne pénètre dans le boîtier à partir de l'intérieur du masque.

17. Fixation selon l'une des revendications 15 ou 16, dans laquelle ledit élément indicateur d'écoulement de gaz est constitué d'une bille.

18. Fixation selon la revendication 17, dans laquelle ladite bille est traitée à l'aide d'un agent antistatique.

19. Fixation selon l'une quelconque des revendications 15 à 18, dans laquelle ledit boîtier permet de voir l'élément indicateur d'écoulement de gaz à travers sa paroi.

20. Fixation selon l'une quelconque des revendications 15 à 19, comportant des moyens (150, 152) pour surveiller automatiquement le déplacement de l'élément indicateur d'écoulement de gaz.

21. Fixation selon la revendication 20, dans laquelle lesdits moyens comportent un émetteur d'infrarouge et un récepteur d'infrarouge disposés sur des côtés opposés du boîtier.

22. Fixation selon l'une quelconque des revendications 15 à 21, dans laquelle ledit boîtier est constitué d'un conduit tubulaire.

23. Fixation selon la revendication 22, dans laquelle ledit conduit a une forme de L, dont la première jambe est adaptée pour communiquer avec le masque et la seconde jambe contient l'élément indicateur d'écoulement de gaz.

24. Fixation selon la revendication 23, dans laquelle ladite première jambe communiquant avec ledit masque peut tourner sur 180 degrés ou plus.

25. Fixation selon l'une des revendications 23 ou 24, comportant des moyens formant tuyau permettant qu'une partie d'alimentation en gaz vers le masque pénètre dans ladite seconde branche.

26. Fixation selon la revendication 25, dans laquelle l'alimentation en gaz vers la seconde branche peut être coupée.

27. Fixation selon l'une des revendications 25 ou 26 qui est ajustable lorsqu'elle est fixée sur le masque pour permettre que la seconde branche s'étende soit vers le haut lorsque du gaz est alimenté vers celle-ci soit vers le bas lorsqu'il n'y en a pas.

28. Fixation selon l'une quelconque des revendications 15 à 27, dans laquelle ladite fixation est jetable après utilisation.
